# EUROPÄISCHE PATENTSCHRIFT

(11) **EP 2 022 429 B1**
(45) Veröffentlichungstag und Bekanntmachung des Hinweises auf die Patenterteilung: **17.05.2017**
(21) Anmeldenummer: 08013074.3
(22) Anmeldetag: 21.07.2008
(51) Int. Cl.: A61B 18/14, A61B 18/12

(54) **Vorrichtung zur Erzeugung von hochfrequenten Strömen, zur kosmetischen und/oder therapeutischen Behandlung von Gewebe mittels Elektroden**
Device for creating high frequency currents for cosmetic and/or therapeutic treatment of tissue with electrodes
Dispositif de production de courants à haute fréquence destiné au traitement cosmétique et/ou thérapeutique de tissus à l'aide d'électrodes

(30) Priorität: 25.07.2007 DE 102007034796
(43) Veröffentlichungstag der Anmeldung: 11.02.2009
(73) Patentinhaber: Wellcomet GmbH, 76137 Karlsruhe (DE)
(72) Erfinder: Kruglikov, Ilja Dr., 76351 Linkenheim-Hochstetten (DE)
(74) Vertreter: Lemcke, Brommer & Partner Patentanwälte Partnerschaft mbB

(56) Entgegenhaltungen:
- EP-A- 1 334 699
- WO-A-2008/012827
- WO-A1-2008/073727
- DE-A1- 19 738 457
- US-A1- 2001 051 803
- US-A1- 2004 162 556
- US-A1- 2007 088 413

## Beschreibung

Die Erfindung betrifft eine Vorrichtung zur Erzeugung von hochfrequenten Strömen, zur kosmetischen und/oder therapeutischen Behandlung von Gewebe mittels Elektroden nach dem Oberbegriff der Ansprüche 1 und 4.

Solche Vorrichtungen finden Anwendung in der Thermotherapie von insbesondere menschlichem Gewebe. Grundprinzip ist hierbei die Erwärmung des Gewebes und hierdurch die Erzeugung von reversiblen oder irreversiblen Veränderungen. Typische Anwendungsgebiete sind beispielsweise die thermische Behandlung von instabilen Gelenkkapseln mittels thermischer Kapsulorrhaphie ("Thermal Collagen Shrinkage"), Thermokeratoplastie, Skin Resurfacing, sowie die Hautfestigung ("Skin Tightening").

All diesen Anwendungen ist gemeinsam, dass mittels Wärme Kollagennetzwerkstrukturen verändert werden.

Die bekanntesten Erwärmungsmethoden für Kollagengewebe sind die Lasererwärmung, sowie die Erwärmung mit hochfrequenten Strömen, welche auch als "radiofrequente Ströme" oder RF-Ströme bezeichnet werden.

Das Grundprinzip bei der Erwärmung mittels hochfrequenter Ströme ist das Einbringen von hochfrequenten Strömen mittels Elektroden in das Gewebe. Typische Vorrichtungen hierzu weisen eine Hochfrequenz-Energiequelle zur Erzeugung der hochfrequenten Ströme auf, sowie ein Elektrodenpaar. Die Elektroden sind mit der Hochfrequenz-Energiequelle verbunden und weisen jeweils eine Kontaktfläche auf, mittels derer sie an das zu beaufschlagende Gewebe angelegt werden. Über die Kontaktflächen wird das Gewebe mit den von der Hochfrequenz-Energiequelle erzeugten Strömen beaufschlagt, sodass aufgrund des Stromflusses durch Joulsche Wärme eine Erwärmung des Gewebes stattfindet.

Problematisch bei solchen Behandlungen ist, dass einerseits eine Schmerzschwelle des Patienten nicht überschritten werden soll und andererseits eine Begrenzung aufgrund der Thermotoleranzgrenze für Gewebeschädigung gegeben ist. Diese Probleme treten insbesondere an der Kontaktfläche der Elektroden mit dem Gewebe auf.

Um diese Grenzen nicht zu überschreiten ist einerseits bekannt, Ströme im Leistungsbereich 40 bis 100 Watt für eine Dauer von 50 bis 1000 Millisekunden oder im niedrigen Sekundenbereich einmalig an das Gewebe abzugeben. Solche Anwendung sind als "High Rate"-Anwendungen bekannt.

Ebenso ist bekannt, das Gewebe mit Leistungen bis 40 Watt für eine Dauer von 10 bis 100 Sekunden zu beaufschlagen, sodass durch solch eine "Low Rate"-Anwendung eine gleiche Wirkung mit niedrigeren Energiedichten und dafür längeren Expositionszeiten erreicht wird.

US 2001/0051803 A1 beschreibt eine Vorrichtung und ein Verfahren zur Ablation mittels mehrfasiger Hochfrequenz, welche einen Elektronenkatheder mit einer Mehrzahl von Elektroden umfasst, wobei die Mehrzahl der Elektroden in einer zweidimensionalen oder dreidimensionalen Elektrodenanordnung angeordnet werden kann. Mittels einer Energieversorgung werden Funkfrequenzspannungen mit individueller Phase für jede der Mehrzahl von Elektroden bereitgestellt.

US 2007/0088413 A1 beschreibt eine Vorrichtung und ein Verfahren zur Behandlung mittels Energiebeaufschlagung eines Gewebes in mehreren unterschiedlich wählbaren Tiefen. Die Vorrichtung umfasst mindestens zwei Elektroden, welche elektrisch voneinander isoliert sind, so dass aufgrund unterschiedlicher Energiebeaufschlagung der beiden Elektroden unterschiedliche Behandlungstiefen erzielbar sind.

US 2004/0162556 A1 beschreibt eine Stromversorgung und eine Steuereinheit sowie ein elektrophysiologisches System, wobei die Stromversorgung in einem bipolaren Modus, einem unipolarem Modus und einem kombinierten bipolar/unipolaren Modus betrieben werden kann.

WO 2008/012827 A2 beschreibt eine Vorrichtung zur nicht-invasiven Behandlung von Hautgewebe. Die Vorrichtung umfasst mindestens eine Einheit zur Erzeugung von elektromagnetischer RF-Energie sowie eine mit der Einheit elektrisch verbindbare Mehrzahl von Elektroden zum Applizieren elektromagnetischer RF-Energie auf die Haut. Mittels einer Controller-Einheit zum Kontrollieren der Applikation von elektromagnetischer Energie ist aus der Mehrzahl von Elektroden mindestens eine Gruppe auswählbar und während des Betriebs der Vorrichtung kann zwischen ausgewählten Gruppen von Elektroden gewechselt werden.

Der vorliegenden Erfindung liegt die Aufgabe zugrunde, die bekannte Vorrichtung zur Erzeugung von hochfrequenten Strömen dahingehend zu verbessern, dass ohne eine wesentliche Erhöhung der thermischen Gewebeoberflächenbelastung an den Kontaktstellen mit den Elektroden eine Erhöhung der in das Gewebe einführbaren Energie und damit der im Gewebe erzeugten Wärme ermöglicht wird. Weiterhin soll die Erhöhung der Wärmeerzeugung erfolgen ohne die Schmerzschwelle des Patienten einerseits oder eine Thermotoleranzgrenze für Gewebeschädigung an den Kontaktstellen andererseits zu überschreiten. Gelöst ist diese Aufgabe durch Vorrichtungen zur Erzeugung von hochfrequenten Strömen, zur kosmetischen und/oder therapeutischen Behandlung von Gewebe mittels Elektroden gemäß Ansprüchen 1 und 4. Vorteilhafte Ausgestaltungen der erfindungsgemäßen Vorrichtungen finden sich in den Ansprüchen 2, 3 und 5 bis 11.

Die erfindungsgemäße Vorrichtung unterscheidet sich von den bekannten Vorrichtungen somit dadurch, dass sie mindestens zwei Paare von Elektroden mit jeweils zwei Elektroden umfasst, wobei alle vier Elektroden mit Ausgängen einer HF-Energiequelle der Vorrichtung verbunden sind. Jedes Paar Elektroden kann somit Gewebe mit von der HF-Energiequelle erzeugten hochfrequenten Strömen beaufschlagen.

Wesentlich ist, dass die vier Elektroden derart angeordnet sind, dass sich der Stromflussweg zwischen dem ersten Elektrodenpaar und der Stromflussweg zwischen dem zweiten Elektrodenpaar zumindest teilweise in einem Überlagerungsbereich in dem Gewebe überlagern. Aufgrund der Anordnung der Elektroden ergibt sich im Gewebe somit ein Überlagerungsbereich, in dem sowohl Strom zwischen der ersten Elektrode des ersten Elektrodenpaares und der zweiten Elektrode des ersten Elektrodenpaares, als auch zwischen der ersten Elektrode des zweiten Elektrodenpaares und der zweiten Elektrode des zweiten Elektrodenpaares fließt.

Hierdurch erfolgt im Überlagerungsbereich eine erhöhte Erwärmung des Gewebes, bei gleichzeitig geringer Belastung des Gewebes an der Kontaktfläche zu den einzelnen Elektroden, so dass eine höhere Erwärmung im Überlagerungsbereich verglichen mit herkömmlichen Vorrichtungen und Verfahren möglich ist, ohne dass die Schmerzgrenze des Patienten oder die Thermotoleranzgrenze der Gewebeoberfläche an den Elektrodenkontaktflächen überschritten wird. Gemäß der Erfindung ist mindestens ein Elektrodenpaar als bipolares Elektrodenpaar ausgebildet.

Ein bipolares Elektrodenpaar weist zwei in etwa identische Elektroden auf, welche eine vergleichsweise kleine Kontaktfläche zum Kontakt mit dem Gewebe besitzen.
Vorteilhafterweise ist die Gewebekontaktfläche kleiner als 2 cm², insbesondere ist eine Gewebekontaktfläche von etwa 5 mm² vorteilhaft.

Bei der Gewebebehandlung mittels einem bipolaren Elektrodenpaar wird der Abstand zwischen den Elektroden typischerweise gering gehalten, im Bereich wenige Millimeter bis wenige Zentimeter. Hierdurch ergibt sich der Vorteil, dass die Stromverteilung im Körper klar lokalisiert ist und verglichen mit anderen Ausführungen der Elektroden sich eine niedrige Belastung der Hautoberfläche ergibt.

Die Wärmeerzeugung findet bei einem bipolaren Elektrodenpaar einerseits direkt unter den Elektroden statt (diese Wärme fließt dann von der Oberfläche in die Tiefe des Gewebes), als auch in einer gewissen Tiefe zwischen den Elektroden aufgrund Joulscher-Wärme, welche durch die Ströme erzeugt wird. Die Tiefe kann durch den Abstand der Kontaktflächen der Elektroden voneinander geregelt werden: Je größer der Abstand zwischen den Elektroden, desto tiefer dringen die erzeugten hochfrequenten Ströme in das Gewebe ein. Ebenso ist bei gleichbleibendem Abstand zwischen den Gewebekontaktflächen die Eindringtiefe von der verwendeten Frequenz abhängig: Je höher die Frequenz, desto näher an der Oberfläche des Gewebes liegt der Hauptfluss der erzeugten Ströme und entsprechend findet auch bei höheren Frequenzen eine Wärmeerzeugung näher an der Oberfläche des Gewebes statt, als bei niedrigeren Frequenzen.

Weiterhin ist es vorteilhaft, wenn mindestens ein Elektrodenpaar als monopolares Elektrodenpaar ausgebildet ist.

Ein monopolares Elektrodenpaar umfasst eine Flächenkontaktelektrode mit einer Gewebekontaktfläche größer als 5 cm². Solch eine Elektrode kann beispielsweise großflächig an das Gewebe angelegt werden. Ebenso sind Handelektroden bekannt, die von einem Menschen mit der Hand umfasst werden können, sodass sich eine großflächige Gewebekontaktfläche ergibt.

Die zweite Elektrode eines monopolaren Elektrodenpaares ist als Kleinkontaktelektrode vergleichbar mit einer Elektrode eines bipolaren Elektrodenpaares ausgebildet, das heißt die Gewebekontaktfläche ist kleiner als 2 cm², vorteilhafterweise beträgt die Gewebekontaktfläche etwa 5 mm².

Bei Anwendung eines monopolaren Elektrodenpaares werden die Ströme durch die Kleinkontaktelektrode in den Körper eingeführt und fließen über die deutlich größere Gewebekontaktfläche der Flächenkontaktelektrode ab, wobei vorteilhafterweise die Flächenkontaktelektrode in großer Entfernung zur der Kleinkontaktelektrode am Gewebe bzw. am Körper angeordnet ist. Das Gewebe in der Nähe der Kleinkontaktelektrode wird durch Joulsche-Wärme erhitzt. Hieraus ergibt sich der Vorteil, dass - verglichen mit einem bipolaren Elektrodenpaar - eine Erwärmung des Gewebes in einer größeren Tiefe stattfindet. Nachteilig ist die starke Belastung des Gewebes an der Gewebekontaktfläche mit der Kleinkontaktelektrode. Ebenso kann die Stromverteilung im Gewebe bzw. im Körper schwer kontrolliert werden.

Die Elektroden der erfindungsgemäßen Vorrichtung können in gängiger Weise ausgeführt sein, insbesondere ist die Ausführung der Elektroden als Metallelektroden zur galvanischen Ankopplung an das Gewebe oder die Ausführung mit isolierten Gewebekontaktflächen zur kapazitiven Ankopplung an das Gewebe denkbar.

Untersuchungen der Anmelderin haben ergeben, dass es insbesondere vorteilhaft ist, wenn das erste Elektrodenpaar der erfindungsgemäßen Vorrichtung als monopolares Elektrodenpaar und das zweite Elektrodenpaar als bipolares Elektrodenpaar ausgebildet ist. In dieser "tripolaren" Ausführung der erfindungsgemäßen Vorrichtung können die Stromflüsse im Gewebe von einem monopolaren Elektrodenpaar mit denen eines bipolaren Elektrodenpaares überlagert werden.

Vorteilhafterweise werden die Elektroden derart angeordnet, dass die Gewebekontaktfläche der Kleinkontaktelektrode des monopolaren Elektrodenpaares sich in etwa mittig zwischen den beiden Gewebekontaktflächen der Elektroden des bipolaren Elektrodenpaares befindet.

Hierdurch kommt es zu einer Überlagerung von hochfrequenten Strömen unter diesen drei Elektroden und somit zu einer stärkeren Erwärmung als bei einer Anwendung durch ein einzelnes monopolares oder bipolares Elektrodenpaar.

Für die Anwendung umfasst die Vorrichtung vorteilhafterweise eine Elektrodenhalterung, in der die Kleinkontaktelektroden des bipolaren Elektrodenpaares und die Kleinkontaktelektrode des monopolaren Elektrodenpaares angeordnet sind. Wie oben beschrieben, ist dabei vorteilhafterweise die Kleinkontaktelektrode des monopolaren Elektrodenpaares in etwa mittig zwischen den beiden Elektroden des bipolaren Elektrodenpaares angeordnet.

Für konstante Anwendungsgebiete ist eine Elektrodenhalterung vorteilhaft, welche einen fixen Abstand zwischen den drei genannten Elektroden aufweist.

Eine vorteilhafte Variabilität ergibt sich durch eine vorteilhafte Ausführung der Elektrodenhalterung, in der die beiden Kleinkontaktelektroden des bipolaren Elektrodenpaares wahlweise in einem Abstand zwischen 0,5 cm und 10 cm, vorzugsweise zwischen 0,5 cm und 5 cm in der Elektrodenhalterung anordbar sind.

Weiterhin ist es vorteilhaft, wenn die Kleinkontaktelektrode des monopolaren Elektrodenpaares als Keramikelektrode ausgeführt ist, um die Hautbelastung weiter zu reduzieren.

Untersuchungen der Anmelderin haben ergeben, dass weiterhin eine Ausführung des ersten und des zweiten Elektrodenpaares jeweils als bipolares Elektrodenpaar vorteilhaft ist. In dieser "tetrapolaren" Ausführung kann das Gewebe somit mittels 4 Kleinkontaktelektroden durch hochfrequente Ströme beaufschlagt werden.

Vorteilhafterweise umfasst die vorgenannte erfindungsgemäße Vorrichtung eine Elektrodenhalterung, in der die vier Elektroden der beiden Elektrodenpaare auf den Eckpunkten eines Rechteckes liegend angeordnet sind, wobei die Elektroden eines Elektrodenpaares auf diagonal gegenüberliegenden Ecken des Rechteckes angeordnet sind. Hierdurch ist sichergestellt, dass in einem Überlagerungsbereich sich die Stromflusslinien beider Elektrodenpaare überlagern und somit eine erhöhte Erwärmung stattfindet, verglichen mit der Erwärmung durch lediglich ein Elektrodenpaar.

Das zu behandelnde Gewebe weist typischerweise Kollagenfasern auf, wobei die elektrischen Leitungseigenschaften parallel und senkrecht zu den Kollagenfasern unterschiedlich sind. Die beschriebene Anordnung von 4 Kleinkontaktelektroden auf den Ecken eines Rechteckes weist weiterhin den Vorteil auf, dass Ströme in unterschiedlichen Richtungen in dem Gewebe fließen und somit bezüglich der Gesamterwärmung des Gewebes eine "Mittelung" der elektrischen Eigenschaften stattfindet, so dass bei dem Anbringen der Elektroden der Verlauf der Kollagenfasern nicht beachtet werden muss.

Insbesondere ist es vorteilhaft, wenn das Rechteck ein Quadrat ist.

Vorteilhafterweise sind die Elektroden derart angeordnet, dass die Elektroden eines Elektrodenpaares zwischen 1 cm und 10 cm voneinander entfernt sind, insbesondere zwischen 2 cm und 5 cm.

Bei Benutzung von tripolaren und tetrapolaren Elektrodenanordnungen ist es vorteilhaft, wenn eine sogenannte "Low-Rate"-Anwendungen mit Leistungen der hochfrequenten Ströme von unter 20 Watt vorgenommen wird. Solche Anwendungen werden typischerweise über eine längere Zeit, das heißt zwischen einer und bis zu 100 Sekunden vorgenommen.

Obwohl bei einer erfindungsgemäßen Vorrichtung mit tripolaren oder tetrapolaren Elektroden in jeden Stromkreis typischerweise nur Leistungen im Bereich von unter 40 Watt angewendet werden und somit die Gewebeoberfläche nicht stark belastet wird, findet in dem Überlagerungsbereich der Ströme der beiden Elektrodenpaare eine Erwärmung statt, die einer "High-Rate"-Anwendung entspricht.

Ebenso ist es mit der erfindungsgemäßen Vorrichtung jedoch auch möglich, Anwendungen mit Leistungen über 40 Watt durchzuführen.

Eine weitere Reduzierung der Oberflächenerwärmung des Gewebes kann erzielt werden, wenn die Vorrichtung mindestens drei Elektrodenpaare umfasst, wobei ein erstes und ein zweites Elektrodenpaar jeweils als bipolares Elektrodenpaar ausgebildet sind und ein drittes Elektrodenpaar als monopolares Elektrodenpaar ausgebildet ist.

In dieser vorteilhaften Ausgestaltung umfasst die Vorrichtung somit sechs Elektroden, von denen fünf als Kleinkontaktelektroden ausgebildet sind.

Vorteilhafterweise sind die fünf Kleinkontaktelektroden derart angeordnet, dass die vier Elektroden der beiden bipolaren Elektrodenpaare wie zuvor beschrieben auf den Eckpunkten eines Rechtecks liegend angeordnet sind und dass weiterhin etwa im Schnittpunkt der Diagonalen des Rechtecks die Kleinkontaktelektrode des monopolaren Elektrodenpaares angeordnet ist.

Alle vorgenannten Elektrodenanordnungen weisen den gemeinsamen Vorteil gegenüber dem Stand der Technik auf, dass sich die Stromflüsse der Elektrodenpaare im Gewebe überlagern und dadurch eine erhöhte Wärmeerzeugung in dem Überlagerungsgebiet erfolgt. Die vorgenannten Vorteil treten auf, wenn alle Elektrodenpaare mit hochfrequenten Strömen der gleichen Frequenz beaufschlagt werden.

Untersuchungen der Anmelderin haben ergeben, dass es insbesondere vorteilhaft ist, die Elektrodenpaare mit hochfrequenten Strömen unterschiedlicher Frequenz zu beaufschlagen, da hierdurch zusätzlich zu den genannten Vorteilen eine Variabilisierung des Überlagerungsbereiches möglich ist. Hier wird durch die Benutzung unterschiedlicher Stromfrequenzen die Erzeugung unterschiedlicher Temperaturprofile im Gewebe ermöglicht.

Insbesondere ist es daher vorteilhaft, die Elektrodenpaare mit Strömen verschiedener Frequenzen zu beaufschlagen, sodass die Energieabsorbtion im Gewebe zusätzlich variabilisiert werden kann:
Hierzu ist die HF-Energiequelle derart ausgeführt ist, dass das erste Paar von Elektroden mit hochfrequenten Strömen einer Frequenz *f₁* und das zweite Paar von Elektroden mit hochfrequenten Strömen einer zu der Frequenz *f₁* unterschiedlichen Frequenz *f₂* beaufschlagbar ist. Sofern weitere Elektrodenpaare vorhanden sind ist es vorteilhaft, diese ebenfalls mit hochfrequenten Strömen mit einer eigenen, von den anderen Elektrodenpaaren unterschiedlichen Frequenz zu beaufschlagen.

Die erfindungsgemäße Vorrichtung unterscheidet sich vom Stand der Technik somit dadurch, dass nicht nur mittels mehrerer Elektrodenpaare eine Beaufschlagung des Gewebes mit hochfrequenten Strömen möglich ist, sondern zusätzlich noch die Frequenz der hochfrequenten Ströme von mindestens zwei Elektrodenpaaren unterschiedlich ist.

Vorteilhafterweise liegen die verwendeten Frequenzen für die beiden Elektrodenpaare im Bereich zwischen 300 KHz bis etwa 15 MHz.

Die von der HF-Energiequelle erzeugten Ströme weisen vorteilhafterweise Frequenzen im Bereich von 0,3 MHz bis 15 MHz, insbesondere im Bereich 1 MHz bis 10 MHz auf.

Vorteilhafterweise ist die HF-Energiequelle derart ausgeführt, dass für jedes angeschlossene Elektrodenpaar die Frequenz in den vorgenannten Bereichen frei wählbar ist, insbesondere ist es vorteilhaft, wenn beide Elektrodenpaare wahlweise auch mit gleicher Frequenz betrieben werden können. Ebenso liegt es im Rahmen der Erfindung, wenn die HF-Energiequelle für jedes Elektrodenpaar jeweils eine eigene HF-Energiequelle umfasst.

Vorzugsweise kann für jedes Elektrodenpaar auch die elektrische Leistung vorgegeben werden, mit der die HF-Energiequelle die Elektroden beaufschlagt. Weiterhin ist es vorteilhaft, wenn die Stromausgänge der HF-Energiequelle jedes Elektrodenpaares von den Stromausgängen der anderen Elektrodenpaare galvanisch getrennt sind.

Weiterhin ist es vorteilhaft, wenn die Elektrodenpaare nicht gleichzeitig mit hochfrequenten Strömen beaufschlagt werden, sondern eine alternierende Beaufschlagung erfolgt. Hierzu ist die HF-Energiequelle vorteilhafterweise derart ausgeführt, dass die angeschlossenen Elektrodenpaare abwechselnd mit hochfrequenten Strömen beaufschlagt werden.

Bei zwei angeschlossenen Elektrodenpaaren erfolgt somit ein wechselseitiges Beaufschlagen, bei mehr als zwei angeschlossenen Elektrodenpaaren ist ein Beaufschlagen der Elektrodenpaare gemäß einer vorgegebenen Reihenfolge sinnvoll, wobei vorzugsweise zu jedem Zeitpunkt nur ein Elektrodenpaar mit hochfrequenten Strömen von der HF-Energiequelle beaufschlagt wird.

Vorteilhafterweise kann eine Zeitdauer vorgegeben werden, innerhalb derer ein Elektrodenpaar mit hochfrequenten Strömen beaufschlagt wird, bevor der Wechsel zu einem nächsten Elektrodenpaar erfolgt. Vorzugsweise erfolgt ein Wechsel alle 100 ms bis 30 s, insbesondere alle 500 ms bis 10 s.

Durch die wechselnde Beaufschlagung der Elektrodenpaare mit hochfrequenten Strömen kann die Oberflächenbelastung des Gewebes noch weiter reduziert und die Anwendungsdauer somit verlängert werden, ohne dem Patienten Schmerzen zuzufügen oder eine Beschädigung des Gewebes zu riskieren.

Beträgt die maximale Belastung beispielsweise der Haut als Gewebeoberfläche durch einen hochfrequenten Strom mit galvanischen Elektroden etwa 0,25 qcm für etwa 10 Sekunden, so kann durch das vorgenannte wechselnde Beaufschlagen der Elektrodenpaare beispielsweise alle 10 Sekunden von einem Elektrodenpaar auf das nächste Elektrodenpaar umgeschaltet werden, um die vorgegebene maximale Belastung nicht zu überschreiten.

Insbesondere ist es vorteilhaft, bei einer tripolaren Vorrichtungen die Elektrodenpaare für unterschiedlich lange Zeitspannen mit Strom zu beaufschlagen: Wie vorhergehend beschrieben weist die Kleinkontaktelektrode eines monopolaren Elektrodenpaares den Nachteil auf, dass an der Kontaktfläche der Kleinkontaktelektrode mit dem Gewebe eine hohe thermische Belastung der Gewebeoberfläche auftritt. Daher ist es vorteilhaft, bei einer tripolaren Vorrichtung das monopolare und das bipolare Elektrodenpaar abwechselnd mit Strom zu beaufschlagen, wobei das bipolare Elektrodenpaar für eine längere Zeitdauer mit Strom beaufschlagt wird als das monopolare Elektrodenpaar. Insbesondere ist es sinnvoll, hierbei ein Verhältnis von etwa 1/3 zu 2/3 zu wählen, d.h. im Wechsel das monopolare Elektrodenpaar z.B. für 5 s mit Strom zu beaufschlagen und anschließend das bipolare Elektrodenpaar für 10 s usw.

Die Energiequelle ist vorzugsweise derart ausgeführt, dass für jedes Elektrodenpaar zusätzlich zu der Frequenz auch die Intensität oder Leistung der hochfrequenten Ströme vorgebbar ist. Hierdurch wird die Variabilität erweitert und für jedes Elektrodenpaar kann gesondert die Intensität der hochfrequenten Ströme und insbesondere die Wärmeentwicklung an der Oberfläche des Gewebes unterhalb der Elektrodenkontaktfläche gewählt werden.

Vorteilhafterweise wird die Anwendung mit weiteren Anwendungsformen kombiniert:
In einer vorteilhaften Ausführungsform weist die Vorrichtung eine Vakuumeinheit auf, welche einen konstanten oder einen pulsierenden Unterdruck erzeugt. Hierzu ist mindestens eine der Elektroden mit einer Vakuumglocke versehen, wobei die Kontaktfläche der Elektrode innerhalb der Vakuumglocke angeordnet ist.

Vorteilhafterweise sind mehrere Elektroden innerhalb der Vakuumglocke angeordnet, insbesondere sämtliche Kleinkontaktelektroden der Vorrichtung.

Die Vakuumglocke ist mit der Vakuumeinheit verbunden, sodass das Gewebe im Bereich der Kontaktfläche der Elektrode mit dem konstanten oder pulsierenden Vakuum zusätzlich zu den hochfrequenten Strömen beaufschlagt werden kann.

Ebenso ist es vorteilhaft, die erfindungsgemäße Vorrichtung mit einer Infrarotanwendung zu kombinieren:
Hierzu weist die Vorrichtung eine Infraroteinheit zur Erzeugung von Infrarotstrahlung auf, welche eine Infrarotquelle und eine Infrarotsonde umfasst.

Die Infrarotsonde ist mit der Infrarotquelle verbunden und derart ausgeführt, dass mittels der Infrarotsonde das Gewebe mit der von der Quelle erzeugten Infrarotstrahlung beaufschlagt werden kann.

Weiterhin ist es vorteilhaft, die Anwendung von hochfrequenten Strömen mit einer Anwendung von Ultraschall zu kombinieren:
Hierzu weist die Vorrichtung eine Ultraschalleinheit auf, welche konstante oder pulsierende Ultraschallwellen erzeugt. Ferner umfasst die Vorrichtung einer mit der Ultraschalleinheit Sonotrode, mittels derer das Gewebe mit den Ultraschallwellen beaufschlagbar ist.

Weitere vorteilhafte Ausgestaltungen der erfindungsgemäßen Vorrichtung werden im Folgenden anhand der Figuren erläutert. Dabei zeigt
- Figur 1: eine erfindungsgemäße Vorrichtung mit zwei Paaren von Elektroden, wobei das erste Elektrodenpaar als monopolares Elektrodenpaar und das zweite Elektrodenpaar als bipolares Elektrodenpaar ausgebildet ist und
- Figur 2: eine erfindungsgemäße Vorrichtung mit zwei Elektrodenpaaren, wobei beide Elektrodenpaare als bipolares Elektrodenpaar ausgebildet sind.

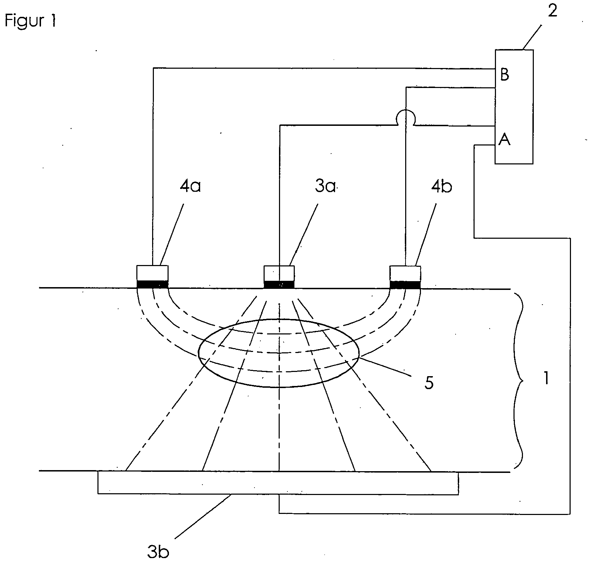

Die in Figur 1 dargestellte Vorrichtung dient zur kosmetischen und/oder therapeutischen Behandlung eines Gewebes 1. Hierzu werden mittels einer Hochfrequenz-Energiequelle 2 hochfrequente Ströme erzeugt, welche mittels der Elektroden dem Gewebe 1 beaufschlagt werden.

Die Vorrichtung weist zwei Elektrodenpaare auf, wobei ein erstes Elektrodenpaar 3a, 3b als monopolares Elektrodenpaar ausgebildet ist und ein zweites Elektrodenpaar 4a, 4b als bipolares Elektrodenpaar ausgebildet ist. Die Vorrichtung in Figur 1 stellt somit eine tripolare Vorrichtung dar.

Entsprechend sind die Elektroden 3a, 4a und 4b als Kleinkontaktelektroden ausgeführt, mit einer Gewebekontaktfläche von etwa 5 mm².

Die Elektrode 3b ist hingegen als Flächenkontaktelektrode ausgeführt, mit einer Gewebekontaktfläche von etwa 40 cm².

Die HF-Energiequelle 2 weist zwei Ausgänge auf, wobei an einen ersten Ausgang A das erste Elektrodenpaar und an einen zweiten Ausgang B das zweite Elektrodenpaar angeschlossen ist. Mittels (nicht dargestellter) Wähltasten der HF-Energiequelle 2 kann für jedes Elektrodenpaar eine Frequenz zwischen 1 MHz und 10 MHz vorgegeben werden, sowie die Leistung, mit welcher das Gewebe durch die Elektroden des jeweiligen Ausgangs beaufschlagt wird. Sämtliche Parameter sind für jedes Elektrodenpaar separat vorgebbar, insbesondere können auch für beide Elektrodenpaare identische Parameter vorgegeben werden.

Das Gewebe 1 ist in Figur 1 im Querschnitt dargestellt. Die gestrichelten Linien in Figur 1 geben Stromflusslinien an, entlang derer die Ströme zwischen den Elektroden 3a und 3b einerseits bzw. 4a und 4b andererseits fließen.

Wesentlich ist, dass in einem Überlagerungsbereich 5 eine Überlagerung der Ströme und damit eine erhöhte Erwärmung des Gewebes 1 stattfindet.

Die Lage und insbesondere die Tiefe des Überlagerungsbereiches 5, das heißt der Abstand des Überlagerungsbereiches 5 von der in Figur 1 oberen Oberfläche des Gewebes 1 kann über den Abstand der Elektroden 4a und 4b zueinander, sowie über die Wahl der Frequenzen der hochfrequenten Ströme beeinflusst werden.

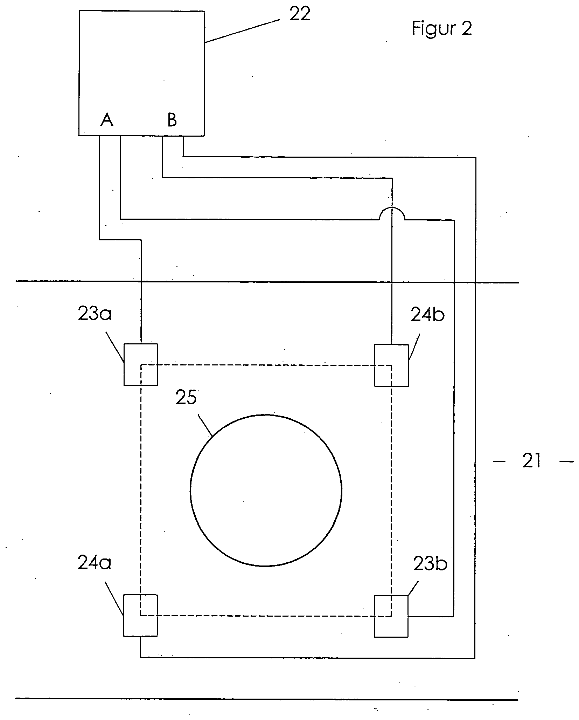

In Figur 2 ist eine erfindungsgemäße Vorrichtung mit zwei Elektrodenpaaren dargestellt, bei der beide Elektrodenpaare (23a und 23b, sowie 24a und 24b) als bipolares Elektrodenpaar ausgebildet sind. Die Vorrichtung in Figur 2 stellt somit eine tetrapolare Vorrichtung dar.

Die Elektrodenpaare sind an Ausgänge A, B einer Hochfrequenz-Energiequelle 22 angeschlossen, welche analog zu der Energiequelle 2 in Figur 1 ausgebildet ist.

Die Elektroden sind derart auf der Oberfläche eines Gewebes 21 angeordnet, dass sie auf den Eckpunkten eines (gestrichelt dargestellten) Quadrates liegen. Das Gewebe 21 ist somit in Figur 2 in Draufsicht dargestellt.

Wesentlich ist, dass sich die Elektroden eines bipolaren Elektrodenpaares jeweils auf dem Quadrat gegenüberliegen. Hierdurch kann durch Wahl der Leistung und der Frequenz, mittels derer ein Elektrodenpaar durch die Energiequelle 22 beaufschlagt wird, die Wärmeentwicklung in einem Überlagerungsbereich 25 sowohl in der Intensität, das heißt in der Höhe der erzeugten Temperaturdifferenz, als auch in der Tiefe, das heißt dem Abstand zur Oberfläche des Gewebes 21 (in Figur 2 somit der Abstand in die Zeichenebene hinein) vorgegeben werden.

## Patentansprüche

1. Vorrichtung zur Erzeugung von hochfrequenten Strömen, zur kosmetischen und/oder therapeutischen Behandlung von Gewebe mittels Elektroden, umfassend
eine Hochfrequenz-Energiequelle (2) zur Erzeugung von hochfrequenten Strömen und mindestens ein Paar Elektroden (3a, 3b; 4a, 4b) zum Beaufschlagen des Gewebes (1) mit hochfrequenten Strömen, wobei die Elektroden mit Ausgängen der HF-Energiequelle verbunden sind, wobei die Vorrichtung mindestens zwei Paare von Elektroden umfasst, wobei alle vier Elektroden (3a, 3b, 4a, 4b) mit Ausgängen der HF-Energiequelle (2) verbunden sind,
wobei das erste Elektrodenpaar als monopolares Elektrodenpaar ausgebildet ist,
umfassend eine Flächenkontaktelektrode (3b) mit einer Gewebekontaktfläche größer 5 cm² und eine Kleinkontaktelektrode (3a) mit einer Gewebekontaktfläche kleiner 2 cm², insbesondere eine Kleinkontaktelektrode mit einer Gewebekontaktfläche von etwa 5 mm² und
das zweite Elektrodenpaar als bipolares Elektrodenpaar ausgebildet ist, umfassend zwei Kleinkontaktelektroden (4a, 4b) mit einer Gewebekontaktfläche kleiner 2 cm², insbesondere zwei Kleinkontaktelektroden mit einer Gewebekontaktfläche von etwa 5 mm², wobei die Vorrichtung derart ausgebildet ist, dass bei Benutzung die Kleinkontaktelektrode (3a) des monoplaren Elektrodenpaares und die Kleinkontaktelektroden (4a, 4b) des bipolare Elektrodenpaares an einer gemeinsamen Seite des Gewebes anordenbar sind
**dadurch gekennzeichnet,**
**dass** die monopolaren und bipolaren Elektrodenpaare an unterschiedliche Ausgänge der HF-Energiequelle angeschlossen und sämtliche Beaufschlagungsparameter für jedes Elektrodenpaar separat vorgebbar sind und dass die Vorrichtung derart ausgebildet ist, dass sich bei Behandlung von Gewebe der Stromflusspfad zwischen dem ersten Elektrodenpaar und der Stromflusspfad zwischen dem zweiten Elektrodenpaar zumindest teilweise in einem Überlagerungsbereich in dem Gewebe überlagern, wobei der Überlagerungsbereich beabstandet zu der gemeinsamen Seite des Gewebes ist, an welcher die Kleinkontaktelektrode (3a) des monoplaren Elektrodenpaares und die Kleinkontaktelektroden (4a, 4b) des bipolare Elektrodenpaares bei Benutzung angeordnet sind.

2. Vorrichtung nach Anspruch 1,
**dadurch gekennzeichnet,**
**dass** die Vorrichtung eine Elektrodenhalterung umfasst, wobei in der Elektrodenhalterung die beiden Kleinkontaktelektroden (4a, 4b) des bipolaren Elektrodenpaares und die Kleinkontaktelektrode (3a) des monopolaren Elektrodenpaares angeordnet sind, insbesondere, dass die Kleinkontaktelektrode des monopolaren Elektrodenpaares (3a) in etwa mittig zwischen den beiden Kleinkontaktelektroden (4a, 4b) des bipolaren Elektrodenpaares angeordnet ist.

3. Vorrichtung nach Anspruch 2,
**dadurch gekennzeichnet,**
**dass** die Elektrodenhalterung derart ausgeführt ist, dass die beiden Kleinkontaktelektroden des bipolaren Elektrodenpaares (4a, 4b) wahlweise in einem Abstand zwischen 0,5 cm und 5 cm in der Elektrodenhalterung anordbar sind.

4. Vorrichtung zur Erzeugung von hochfrequenten Strömen, zur kosmetischen und/oder therapeutischen Behandlung von Gewebe mittels Elektroden, umfassend
eine Hochfrequenz-Energiequelle (22) zur Erzeugung von hochfrequenten Strömen und mindestens ein Paar Elektroden (23a, 23b; 24a, 24b) zum Beaufschlagen des Gewebes (21) mit hochfrequenten Strömen, wobei die Elektroden mit Ausgängen der HF-Energiequelle (22) verbunden sind,
wobei die Vorrichtung mindestens zwei Paare von Elektroden umfasst,
wobei alle vier Elektroden (23a, 23b, 24a, 24b) mit Ausgängen der HF-Energiequelle (22) verbunden sind,
wobei das erste Elektrodenpaar und das zweite Elektrodenpaar jeweils als bipolares Elektrodenpaar ausgebildet ist,
wobei jedes Elektrodenpaar zwei Kleinkontaktelektroden (23a, 23b, 24a, 24b) mit einer Gewebekontaktfläche kleiner 2 cm² umfasst, insbesondere zwei Kleinkontaktelektroden mit einer Gewebekontaktfläche von etwa 5 mm² umfasst, wobei die Vorrichtung derart ausgebildet ist, dass bei Benutzung die vier Elektroden (23a, 23b, 24a, 24b) der beiden bipolaren Elektrodenpaare an einer gemeinsamen Seite des Gewebes anordenbar sind
**dadurch gekennzeichnet,**
**dass** die beiden bipolaren Elektrodenpaare an unterschiedliche Ausgänge der HF-Energiequelle angeschlossen und sämtliche Beaufschlagungsparameter für jedes Elektrodenpaar separat vorgebbar sind und dass die Vorrichtung derart ausgebildet ist, dass sich bei Behandlung von Gewebe der Stromflusspfad zwischen dem ersten Elektrodenpaar und der Stromflusspfad zwischen dem zweiten Elektrodenpaar zumindest teilweise in einem Überlagerungsbereich in dem Gewebe überlagern, wobei der Überlagerungsbereich beabstandet zu der gemeinsamen Seite des Gewebes ist, an welcher die die beiden Elektrodenpaare bei Benutzung angeordnet sind.

5. Vorrichtung nach Anspruch 4,
**dadurch gekennzeichnet,**
**dass** die Vorrichtung eine Elektrodenhalterung umfasst und die vier Elektroden (23a, 23b, 24a, 24b) der beiden Elektrodenpaare in der Elektrodenhalterung auf den Eckpunkten eines Rechteckes liegend angeordnet sind, wobei die Elektroden eines Elektrodenpaares auf diagonal gegenüberliegenden Ecken des Rechteckes angeordnet sind, insbesondere, dass das Rechteck ein Quadrat ist.

6. Vorrichtung nach einem der Ansprüche 4 bis 5,
**dadurch gekennzeichnet,**
**dass** die Vorrichtung mindestens drei Elektrodenpaare umfasst, wobei ein erstes und ein zweites Elektrodenpaar jeweils als bipolares Elektrodenpaar gemäß einem der Ansprüche 4 oder 5 ausgebildet sind und ein drittes Elektrodenpaar als monopolares Elektrodenpaar gemäß Anspruch 1 ausgebildet ist.

7. Vorrichtung nach Anspruch 6,
**dadurch gekennzeichnet,**
**dass** die Kleinkontaktelektrode des dritten Elektrodenpaares etwa im Schnittpunkt der Diagonalen des Rechteckes angeordnet ist.

8. Vorrichtung nach einem der vorangegangenen Ansprüche 1 bis 7,
**dadurch gekennzeichnet,**
**dass** die HF-Energiequelle (2, 22) derart ausgeführt ist, dass das erste Paar von Elektroden (3a, 3b; 23a, 23b) mit hochfrequenten Strömen einer Frequenz *f₁* und das zweite Paar von Elektroden (4a, 4b; 24a, 24b) mit hochfrequenten Strömen einer zu der Frequenz *f₁* unterschiedlichen Frequenz *f₂* beaufschlagbar ist.

9. Vorrichtung nach einem der vorangegangenen Ansprüche,
**dadurch gekennzeichnet,**
**dass** für die von der HF-Energiequelle (2, 22) erzeugten Ströme Frequenzen im Bereich 0,3 MHz bis 15 MHz, insbesondere im Bereich 1 MHz bis 10 MHz auswählbar sind.

10. Vorrichtung nach einem der vorangegangenen Ansprüche,
**dadurch gekennzeichnet,**
**dass** die HF-Energiequelle (2, 22) derart ausgeführt ist, dass die angeschlossenen Elektrodenpaare abwechselnd mit hochfrequenten Strömen beaufschlagt werden, insbesondere, dass eine Zeitdauer vorgebbar ist, innerhalb derer ein Elektrodenpaar mit hochfrequenten Strömen beaufschlagt wird, bevor der Wechsel zu einem nächsten Elektrodenpaar erfolgt.

11. Vorrichtung nach einem der vorangegangenen Ansprüche,
**dadurch gekennzeichnet.**
**dass** die HF-Energiequelle (2, 22) derart ausgeführt ist, dass für jedes Elektrodenpaar zusätzlich zu der Frequenz auch die Intensität der hochfrequenten Ströme vorgebbar ist.

## Claims

1. Apparatus for producing high frequency currents for the cosmetic and/or therapeutic treatment of tissue by means of electrodes, including a high frequency energy source (2) for producing high frequency currents and at least one pair of electrodes (3a, 3b; 4a, 4b) for applying high frequency currents to the tissue (1), wherein the electrodes are connected to outputs of the HF energy source, wherein the apparatus includes at least two pairs of electrodes, wherein all four electrodes (3a, 3b, 4a, 4b) are connected to outputs of the HF energy source (2), wherein the first pair of electrodes is constructed as a monopolar pair of electrodes, including a surface contact electrode (3b) with a tissue contact area larger than 5 cm² and a small contact electrode (3a) with a tissue contact area smaller than 2 cm², particularly a small contact electrode with a tissue contact area of about 5 mm² and the second pair of electrodes is constructed as a bipolar pair of electrodes including two small contact electrodes (4a, 4b) with a tissue contact area smaller than 2 cm², particularly two small contact electrodes with a tissue contact area of about 5 mm², wherein the apparatus is so constructed that, in use, the small contact electrode (3a) of the monopolar pair of electrodes and the small contact electrodes (4a, 4b) of the bipolar pair of electrodes may be arranged on a common side of the tissue, **characterised in that** the monopolar and bipolar pairs of electrodes are connected to different outputs of the HF energy source and all the application parameters for each pair of electrodes may be set separately and that the apparatus is so constructed that, when treating tissue, the current flow path between the first pair of electrodes and the current flow path between the second pair of electrodes at least partially overlap in the tissue in an overlap region, wherein the overlap region is spaced from the common side of the tissue, on which the small contact electrode (3a) of the monopolar pair of electrodes and the small contact electrodes (4a, 4b) of the bipolar pair of electrodes are arranged, in use.

2. Apparatus as claimed in Claim 1, **characterised in that** the apparatus includes an electrode holder, wherein the two small contact electrodes (4a, 4b) of the bipolar pair of electrodes and the small contact electrode (3a) of the monopolar pair of electrodes are arranged in the electrode holder, particularly that the small contact electrode of the monopolar pair of electrodes (3a) is arranged approximately centrally between the two small contact electrodes (4a) of the bipolar pair of electrodes.

3. Apparatus as claimed in Claim 2, **characterised in that** the electrode holder is constructed so that the two small contact electrodes of the bipolar pair of electrodes (4a, 4b) may be arranged selectively at a distance between 0.5 cm and 5 cm in the electrode holder.

4. Apparatus for producing high frequency currents for the cosmetic and/or therapeutic treatment of tissue by means of electrodes, including a high frequency energy source (22) for producing high frequency currents and at least one pair of electrodes (23a, 23b; 24a, 24b) for applying high frequency currents to the tissue (21), wherein the electrodes are connected to outputs of the HF energy source (22), wherein the apparatus includes at least two pairs of electrodes, wherein all four electrodes (23a, 23b, 24a, 24b) are connected to outputs of the HF energy source (22), wherein the first pair of electrodes and the second pair of electrodes are each constructed as a bipolar pair of electrodes, wherein each pair of electrodes includes two small contact electrodes (23a, 23b, 24a, 24b) with a tissue contact area smaller than 2 cm², and particularly includes two small contact electrodes with a tissue contact area of about 5 mm², wherein the apparatus is so constructed that, in use, the four electrodes (23a, 23b, 24a, 24b) of the two bipolar pairs of electrodes may be arranged on a common side of the tissue, **characterised in that** the two bipolar pairs of electrodes are connected to different outputs of the HF energy source and all the application parameters for each pair of electrodes may be set separately and that the apparatus is so constructed that, when treating tissue, the current flow path between the first pair of electrodes and the current flow path between the second pair of electrodes overlap at least partially in an overlap region in the tissue, wherein the overlap region is spaced from the common side of the tissue, on which the two pairs of electrodes are arranged, in use.

5. Apparatus as claimed in Claim 4, **characterised in that** the apparatus includes an electrode holder and the four electrodes (23a, 23b, 24a, 24b) of the two pairs of electrodes are arranged in the electrode holder lying on the corners of a rectangle, wherein the electrodes of one pair of electrodes are arranged at diagonally opposed corners of the rectangle, particularly that the rectangle is a square.

6. Apparatus as claimed in one of Claims 4 to 5, **characterised in that** the apparatus includes at least three pairs of electrodes, wherein a first and a second pair of electrodes are each constructed as a bipolar pair of electrodes in accordance with one of Claims 4 or 5 and a third pair of electrodes is constructed as a monopolar pair of electrodes in accordance with Claim 1.

7. Apparatus as claimed in Claim 6, **characterised in that** the small contact electrode of the third pair of electrodes is arranged approximately at the intersection of the diagonals of the rectangle.

8. Apparatus as claimed in the preceding Claims 1 to 7, **characterised in that** the HF energy source (2, 22) is constructed so that the first pair of electrodes (3a, 3b; 23a, 23b) may be acted upon with high frequency currents of a frequency *f₁* and the second pair of electrodes (4a, 4b; 24a, 24b) may be acted upon by high frequency currents of a frequency *f₂* different to the frequency *f₁*.

9. Apparatus as claimed in one of the preceding claims, **characterised in that** frequencies in the range of 0.3 MHz to 15 MHz, particularly in the region of 1 MHz to 10 MHz are selectable for the currents produced by the HF energy source (2, 22).

10. Apparatus as claimed in one of the preceding claims, **characterised in that** the HF energy source (2, 22) is so constructed that the connected pairs of electrodes are acted upon alternately with high frequency currents, particularly that a period of time may be set, within which a pair of electrodes is acted upon with high frequency currents before the change to a next pair of electrodes occurs.

11. Apparatus as claimed in one of the preceding claims, **characterised in that** the HF energy source (2, 22) is so constructed that for each pair of electrodes in addition to the frequency the intensity of the high frequency currents may also be set.

## Revendications

1. Dispositif de production de courants à haute fréquence destiné au traitement cosmétique et/ou thérapeutique d'un tissu à l'aide d'électrodes, comprenant une source d'énergie à haute fréquence (2) pour produire des courants à haute fréquence et au moins une paire d'électrodes (3a, 3b ; 4a, 4b) pour exposer le tissu (1) à des courants à haute fréquence, les électrodes étant reliées à des sorties de la source d'énergie HF, le dispositif comprenant au moins deux paires d'électrodes, toutes les quatre électrodes (3a, 3b, 4a, 4b) étant reliées à des sorties de la source d'énergie HF (2),
la première paire d'électrodes étant réalisée sous la forme d'une paire d'électrodes monopolaires, comprenant une électrode à contact surfacique (3b) avec une surface de contact avec le tissu supérieure à 5 cm² et une électrode à faible surface de contact (3a) ayant une surface de contact avec le tissu inférieure à 2 cm², en particulier une électrode à faible surface de contact ayant une surface de contact avec le tissu d'environ 5 mm², et
la deuxième paire d'électrodes étant réalisée sous la forme d'une paire d'électrodes bipolaires, comprenant deux électrodes à faible surface de contact (4a, 4b) ayant une surface de contact avec le tissu inférieure à 2 cm², en particulier deux électrodes à faible surface de contact ayant une surface de contact avec le tissu d'environ 5 mm²,
le dispositif étant réalisé de telle façon que, lors de l'utilisation, l'électrode à faible surface de contact (3a) de la paire d'électrodes monopolaires et les électrodes à faible surface de contact (4a, 4b) de la paire d'électrodes bipolaires puissent être disposées sur un côté commun du tissu,
**caractérisé en ce**
**que** les paires d'électrodes monopolaires et bipolaires sont raccordées à des sorties différentes de la source d'énergie HF et tous les paramètres d'exposition peuvent être prescrits séparément pour chaque paire d'électrodes et
**que** le dispositif est réalisé de telle façon que, lors du traitement de tissu, le trajet de courant entre la première paire d'électrodes et le trajet de courant entre la deuxième paire d'électrodes se superposent au moins partiellement dans une zone de superposition dans le tissu, la zone de superposition étant à distance du côté commun du tissu sur lequel l'électrode à faible surface de contact (3a) de la paire d'électrodes monopolaires et les électrodes à faible surface de contact (4a, 4b) de la paire d'électrodes bipolaires sont disposées lors de l'utilisation.

2. Dispositif selon la revendication 1,
**caractérisé en ce**
**que** le dispositif comprend un porte-électrodes, les deux électrodes à faible surface de contact (4a, 4b) de la paire d'électrodes bipolaires et l'électrode à faible surface de contact (3a) de la paire d'électrodes monopolaires étant disposées dans le porte-électrodes, en particulier que l'électrode à faible surface de contact de la paire d'électrodes monopolaires (3a) est disposée à peu près au milieu entre les deux électrodes à faible surface de contact (4a, 4b) de la paire d'électrodes bipolaires.

3. Dispositif selon la revendication 2,
**caractérisé en ce**
**que** le porte-électrodes est réalisé de telle façon que les deux électrodes à faible surface de contact de la paire d'électrodes bipolaires (4a, 4b) puissent être disposées dans le porte-électrodes au choix à une distance comprise entre 0,5 cm et 5 cm.

4. Dispositif de production de courants à haute fréquence destiné au traitement cosmétique et/ou thérapeutique d'un tissu à l'aide d'électrodes, comprenant une source d'énergie à haute fréquence (22) pour produire des courants à haute fréquence et au moins une paire d'électrodes (23a, 23b ; 24a, 24b) pour exposer le tissu (21) à des courants à haute fréquence, les électrodes étant reliées à des sorties de la source d'énergie HF (22),
le dispositif comprenant au moins deux paires d'électrodes, toutes les quatre électrodes (23a, 23b, 24a, 24b) étant reliées à des sorties de la source d'énergie HF (22),
la première paire d'électrodes et la deuxième paire d'électrodes étant réalisées chacune sous la forme d'une paire d'électrodes bipolaires,
chaque paire d'électrodes comprenant deux électrodes à faible surface de contact (23a, 23b, 24a, 24b) ayant une surface de contact avec le tissu inférieure à 2 cm², en particulier deux électrodes à faible surface de contact ayant une surface de contact avec le tissu d'environ 5 mm²,
le dispositif étant réalisé de telle façon que, lors de l'utilisation, les quatre électrodes (23a, 23b, 24a, 24b) des deux paires d'électrodes bipolaires puissent être disposées sur un côté commun du tissu,
**caractérisé en ce**
**que** les deux paires d'électrodes bipolaires sont raccordées à des sorties différentes de la source d'énergie HF et tous les paramètres d'exposition peuvent être prescrits séparément pour chaque paire d'électrodes et
**que** le dispositif est réalisé de telle façon que, lors du traitement de tissu, le trajet de courant entre la première paire d'électrodes et le trajet de courant entre la deuxième paire d'électrodes se superposent au moins partiellement dans une zone de superposition dans le tissu, la zone de superposition étant à distance du côté commun du tissu sur lequel les deux paires d'électrodes sont disposées lors de l'utilisation.

5. Dispositif selon la revendication 4,
**caractérisé en ce**
**que** le dispositif comprend un porte-électrodes
et les quatre électrodes (23a, 23b, 24a, 24b) des deux paires d'électrodes sont disposées dans le porte-électrodes aux sommets d'un rectangle, les électrodes d'une paire d'électrodes étant disposées à des sommets diagonalement opposés du rectangle, en particulier que le rectangle est un carré.

6. Dispositif selon l'une des revendications 4 à 5,
**caractérisé en ce**
**que** le dispositif comprend au moins trois paires d'électrodes, une première et une deuxième paire d'électrodes étant chaque fois réalisées sous la forme d'une paire d'électrodes bipolaires selon l'une des revendications 4 ou 5 et une troisième paire d'électrodes étant réalisée sous la forme d'une paire d'électrodes monopolaires selon la revendication 1.

7. Dispositif selon la revendication 6,
**caractérisé en ce**
**que** l'électrode à faible surface de contact de la troisième paire d'électrodes est disposée à peu près au point d'intersection des diagonales du rectangle.

8. Dispositif selon l'une des revendications précédentes 1 à 7,
**caractérisé en ce**
**que** la source d'énergie HF (2, 22) est réalisée de telle façon que la première paire d'électrodes (3a, 3b ; 23a, 23b) puisse être soumise à des courants à haute fréquence d'une fréquence *ƒ₁* et la deuxième paire d'électrodes (4a, 4b ; 24a, 24b) à des courants à haute fréquence d'une fréquence *ƒ₂* différente de la fréquence *ƒ₁.*

9. Dispositif selon l'une des revendications précédentes,
**caractérisé en ce**
**que** des fréquences dans la plage de 0,3 MHz à 15 MHz, en particulier dans la plage de 1 MHz à 10 MHz, peuvent être sélectionnées pour les courants produits par la source d'énergie HF (2, 22).

10. Dispositif selon l'une des revendications précédentes,
**caractérisé en ce**
**que** la source d'énergie HF (2, 22) est réalisée de telle façon que les paires d'électrodes raccordées soient soumises alternativement à des courants à haute fréquence, en particulier qu'il soit possible de prescrire une durée pendant laquelle une paire d'électrodes est soumise à des courants à haute fréquence, avant que le passage à une paire d'électrodes suivante ait lieu.

11. Dispositif selon l'une des revendications précédentes,
**caractérisé en ce**
**que** la source d'énergie HF (2, 22) est réalisée de telle façon que, pour chaque paire d'électrodes, en plus de la fréquence, l'intensité des courants à haute fréquence puisse également être prescrite.
